# EUROPEAN PATENT APPLICATION

(11) **EP 2 223 690 A1**
(43) Date of publication of application: **01.09.2010**
(21) Application number: 08863974.5
(22) Date of filing: 18.12.2008
(51) Int. Cl.: A61K 31/426, A61K 31/18, A61P 13/02, A61P 13/08, A61P 43/00

(54) **PHARMACEUTICAL COMPOSITION FOR AMELIORATING LOWER URINARY TRACT SYMPTOM**

(30) Priority: 21.12.2007 JP 2007330434
(71) Applicant: Astellas Pharma Inc., Tokyo 103-8411 (JP)
(72) Inventor: SUZUKI, Masanori, Tokyo 103-8411 (JP); UKAI, Masashi, Tokyo 103-8411 (JP); OHTAKE, Akiyoshi, Tokyo 103-8411 (JP); KODA, Mai, Tokyo 103-8411 (JP)
(74) Representative: HOFFMANN EITLE
(86) International application number: PCT/JP2008/073070
(87) International publication number: WO 2009/081837

(57) **Abstract**

[Problems] To provide a pharmaceutical composition which is useful as a pharmaceutical composition for improving lower urinary tract symptoms.

[Means for solution] A pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl)amino]propyl}-2-methoxybenzene-1-sulfonamide or a pharmaceutically acceptable salt thereof, as active ingredients, particularly a pharmaceutical composition for improving lower urinary tract symptoms, more specifically a pharmaceutical composition for improving lower urinary tract symptoms associated with benign prostatic hyperplasia.

## Description

### Technical Field

The present invention relates to a pharmaceutical, in particular, a pharmaceutical composition useful as a pharmaceutical composition for improving lower urinary tract symptoms and, in an embodiment, lower urinary tract symptoms associated with benign prostatic hyperplasia, particularly, a pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl)amino]propyl}-2-methoxybenzene-1-sulfonamide or a pharmaceutically acceptable salt thereof, as active ingredients. Further, the present invention relates to a combined use and a combination therapy of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{([2-(2-ethoxyphenoxy)ethyl)amino]propyl}-2-methoxybenzene-1-sulfonamide or a pharmaceutically acceptable salt thereof, for improving lower urinary tract symptoms and, in an embodiment, lower urinary tract symptoms associated with benign prostatic hyperplasia.

### Background Art

The prostate is an organ situated between the bladder neck and the external urethral sphincter, which surrounds the urethra in males. A prostate tumor appearing in benign prostatic hyperplasia is a benign tumor occurring in the transitional zone (inner gland) of the prostate, and growth thereof depends on a male hormone (androgen), but details of the mechanism have not yet been demonstrated.
It has been considered that direct urethral compression due to hypertrophy of adenoma (mechanical obstruction) and elevation of intraurethral pressure due to overcontraction of the prostate or the urethra via the sympathetic nerve or the like (functional obstruction) are primarily involved in the pathogenesis of benign prostatic hyperplasia. There is a report that hypertrophy of prostate defined histologically increases with the age, and is confirmed in 90% of males of the age of 70 to 80 years.
It is considered that the voiding dysfunction associated with benign prostatic hyperplasia occurs due to excessive contraction of the prostate and the urethra, in addition to hypertrophy of prostate as mentioned above, and its clinical symptoms include the appearance of various lower urinary tract symptoms such as voiding symptoms (weak stream, fractionated urination, hesitancy, straining, and the like), storage symptoms (urinary frequency, nocturia, urinary urgency, and the like) and postmicturition symptoms (feeling of residual urine, postmicturition dribble, and the like). Benign prostatic hyperplasia is defined generically referring to these symptoms. For this reason, severity of the disease in benign prostatic hyperplasia is judged using wide-ranging evaluation such as urodynamic study, prostate weight, and international prostate symptom score (I-PSS).

An α₁-adrenoceptor antagonist for the purpose of alleviation of functional obstruction is currently well known as a representative medication therapy for benign prostatic hyperplasia and is already used worldwide as well as in Japan, as the first-line agent for voiding dysfunction associated with benign prostatic hyperplasia. As a mechanism for which the α₁-adrenoceptor antagonist is therapeutically successful, it is mentioned that the α₁-adrenoceptor mediates the contraction of prostatic and urethral smooth muscles.
The α₁-adrenoceptor antagonists are used as agents for the treatment of voiding dysfunction associated with benign prostatic hyperplasia, and confirmed to improve voiding symptoms from its mechanism. The α₁-adrenoceptor antagonist has been confirmed to improve storage symptoms as well as voiding symptoms, however, the improving effects on storage symptoms are not satisfactory (Non-Patent Document 1).

Meanwhile, the prostate is a target organ of a male hormone, androgen, and androgen is closely related to the pathogenesis and progression of benign prostatic hyperplasia. As a drug for inhibiting such an action of androgen, chlormadinone acetate and allylestrenol are therapeutically used in Japan. Further, in Western countries, finasteride and dutasteride, which are drugs inhibiting an enzyme that converts testosterone to dihydrotestosterone in prostate (5α-reductase), have been therapeutically used for the purpose of the improvement of voiding dysfunction due to the reduction of the prostate.

In addition, it has been reported that there are male patients who complain lower urinary tract symptoms irrespective of hypertrophy of prostate, and there are also patients complaining lower urinary tract symptoms even among female patients, and these are referred to also as male lower urinary tract symptoms and female lower urinary tract symptoms, respectively.

Under these circumstances, there is a document which discloses the treatment of bladder dysfunction by a pharmaceutical composition containing an α₁-adrenoceptor antagonist and a β₃-adrenoceptor agonist (Patent Document 1).
In this document, numerous compounds are respectively cited for the α₁-adrenoceptor antagonist and the β₃-adrenoceptor agonist to be combined. Also with regard to the combination of (R)-2-(2-aminothiazol-4-yl)-4'-(2-[(2-hydroxy-2-phenylethyl)amino]ethyl)acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, according to the present invention, such a combination can be found among mere citation of numerous combinations between numerous compounds. However, it cannot be said that the combination of the present invention is substantially disclosed, since not all of such numerous combinations are described to exhibit the effects, and it is merely mechanical and comprehensive citation of the combinations. In addition, there is no disclosure showing that the effects were actually confirmed for the combination of the present invention.

There is a disclosure that (R)-2-(2-aminothiazol-4-yl)-4'-(2-[(2-hydroxy-2-phenylethyl)amino]ethyl}acetanilide has a β₃-adrenoceptor agonistic activity, and is useful as an agent for the treatment of overactive bladder, as a single agent (Patent Document 2). Further, this compound has a chemical structure below and is also known as YM178.

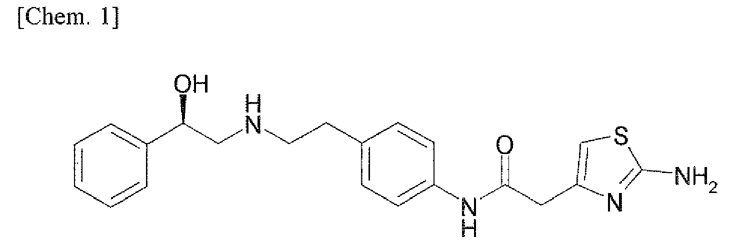

It is known that (R)-5-(2-{[2-(2-ethoxyphenoxy)ethyl]amino}propyl)-2-methoxybenzene-1-sulfonamide has an α₁-adrenoceptor antagonistic activity, and is useful as an agent for the treatment of voiding dysfunction associated with benign prostatic hyperplasia, as a single agent. Further, this compound has a chemical structure below, and is also known as tamsulosin or YM617.

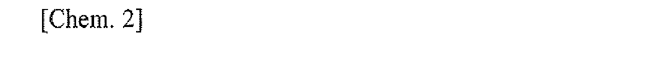

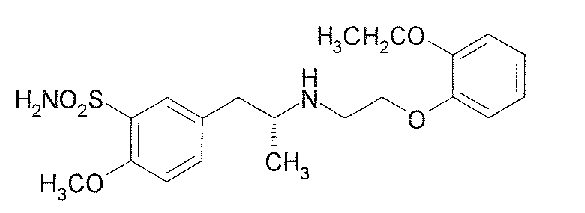

Patent Document 1: Pamphlet of International Publication No. WO 2005/042021
Patent Document 2: Pamphlet of International Publication No. WO 2004/041276
Non-Patent Document 1: BJU International, Vol. 94, p. 817, 2004

### Disclosure of the Invention

### Problem that the Invention is to Solve

A pharmaceutical composition containing (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, specifically a pharmaceutical composition for improving lower urinary tract symptoms, and more specifically a pharmaceutical composition for improving lower urinary tract symptoms associated with benign prostatic hyperplasia, are provided.
Further, a combined use and a combination therapy of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof for improving lower urinary tract symptoms, specifically lower urinary tract symptoms associated with benign prostatic hyperplasia, are provided.

### Means for Solving the Problem

As a result of studies on pharmaceutical compositions for improving lower urinary tract symptoms, containing an α₁-adrenoceptor antagonist and a β₃-adrenoceptor agonist, the present inventors have found that particularly a combination of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof exhibits excellent improving effects on lower urinary tract symptoms, and thus completed the present invention.

That is, the present invention relates to;
1. a pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-(2-{[2-ethoxyphenoxy)ethyl]amino}propyl)-2-methoxybenzene-1-sulfonamide or a pharmaceutically acceptable salt thereof, as active ingredients;
2. the pharmaceutical composition of [1], wherein the (R)-2-(2-aminothiazol-4-yl)-4'-{2-[(2-hydroxy-2-phenylethyl)amino]ethyl}acetanilide or a pharmaceutically acceptable salt thereof is (R)-2-(2-aminothiazol-4-yl)-4'-{2-[(2-hydroxy-2-phenylethyl)amino]ethyl}acetanilide;
3.the pharmaceutical composition of [1] or [2], wherein the (R)-5-(2-{[2-(2-ethoxyphenoxy)ethyl]amino}propyl)-2-methoxybenzene-1-sulfonamide or a pharmaceutically acceptable salt thereof is (R)-5-(2-{[2-(2-ethoxyphenoxy)ethyl]amino}propyl)-2-methoxybenzene-1-sulfonamide hydrochloride;
4. the pharmaceutical composition of any one of [1] to [3], further comprising a pharmaceutically acceptable excipient;
5. a pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof at an amount of 10 mg to 100 mg in terms of (R)-2-(2-aminothiazol-4-yl)-4'-{2-[(2-hydroxy-2-phenylethyl)amino]ethyl}acetanilide and (R)-5-(2-{[2-(2-ethoxyphenoxy)ethyl]amino}propyl)-2-methoxybenzene-1-sulfonamide or a pharmaceutically acceptable salt thereof at an amount of 0.1 mg to 0.8 mg in terms of (R)-5-(2-{[2-(2-ethoxyphenoxy)ethyl]amino}propyl)-2-methoxybenzene-1-sulfonamide hydrochloride, as active ingredients;
6. the pharmaceutical composition of [5], comprising 10 mg to 100 mg of (R)-2-(2-aminothiazol-4-yl)-4'-{2-[(2-hydroxy-2-phenylethyl)amino]ethyl}acetanilide and 0.1 mg to 0.8 mg of (R)-5-(2-{[2-(2-ethoxyphenoxy)ethyl]amino)propyl)-2-methoxybenzene-1-sulfonamide hydrochloride;
7. the pharmaceutical composition of any one of [1] to [6], which is a pharmaceutical composition for improving lower urinary tract symptoms;
8. the pharmaceutical composition of any one of [1] to [7], which is a preparation for oral administration;
9. a method for improving lower urinary tract symptoms, comprising administering effective amounts of each of (R)-2-(2-aminothiazol-4-yl)-4'-{2-[(2-hydroxy-2-phenylethyl)amino]ethyl}acetanilide or a pharmaceutically acceptable salt thereof and (R)-5-(2-([2-(2-ethoxyphenoxy)ethyl]amino}propyl)-2-methoxybenzene-1-sulfonamide or a pharmaceutically acceptable salt thereof;
10. the method for improving of [9], comprising administering effective amounts of each of (R)-2-(2-aminothiazol-4-yl)-4'-{2-[(2-hydroxy-2-phenylethyl)amino]ethyl}acetam!ide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, simultaneously or at a time interval;
11. use of (R)-5-(2-{[2-(2-ethoxyphenoxy)ethyl]amino}propyl)-2-methoxybenzene-1-sulfonamide or a pharmaceutically acceptable salt thereof, for the manufacture of a pharmaceutical composition for improving lower urinary tract symptoms, which is used in combination with (R)-2-(2-aminothiazol-4-yl)-4'-{2-[(2-hydroxy-2-phenylethyl)amino]ethyl}acetanilide or a pharmaceutically acceptable salt thereof;
12. use of (R)-2-(2-aminothiazol-4-yl)-4'-{2-[(2-hydroxy-2-phenylethyl)amino]ethyl}acetanilide or a pharmaceutically acceptable salt thereof, for the manufacture of a pharmaceutical composition for improving lower urinary tract symptoms, which is used in combination with (R)-5-(2-{[2-(2-ethoxyphenoxy)ethyl]amino}propyl)-2-methoxybenzene-1-sulfonamide or a pharmaceutically acceptable salt thereof;
13. a pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof as an active ingredient, which is used in combination with (R)-5-(2-{[2-(2-ethoxyphenoxy)ethyl]amino}propyl)-2-methoxybenzene-1-sulfonamide or a pharmaceutically acceptable salt thereof;
14. a pharmaceutical composition comprising (R)-5-(2-{[2-(2-ethoxyphenoxy)ethyl]amino}propyl)-2-methoxybenzene-1-sulfonamide or a pharmaceutically acceptable salt thereof as an active ingredient, which is used in combination with (R)-2-(2-aminothiazol-4-yl)-4'-{2-[(2-hydroxy-2-phenylethyl)amino]ethyl}acetanilide or a pharmaceutically acceptable salt thereof

Further, the present invention relates to a pharmaceutical composition for improving lower urinary tract symptoms containing (R)-2-(2-aminothiazol-4-yl)'4'-{2-[(2-hydroxy-2-phenylethyl)amino]ethyl}acetanilide or a pharmaceutically acceptable salt thereof and (R)-5-(2-([2-(2-ethoxyphenoxy)ethyl]amino)propyl)-2-methoxybenzene-1-sulfonamide or a pharmaceutically acceptable salt thereof, that is, an agent for improving lower urinary tract symptoms, containing (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof.
Further, the present invention relates to a use of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, for the manufacture of a pharmaceutical composition for improving lower urinary tract symptoms, and a method for improving lower urinary tract symptoms, comprising administering to a patient effective amounts of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof.

### Effects of the Invention

A pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof as active ingredients can be used as a pharmaceutical composition for improving lower urinary tract symptoms.

### Brief Description of Drawings

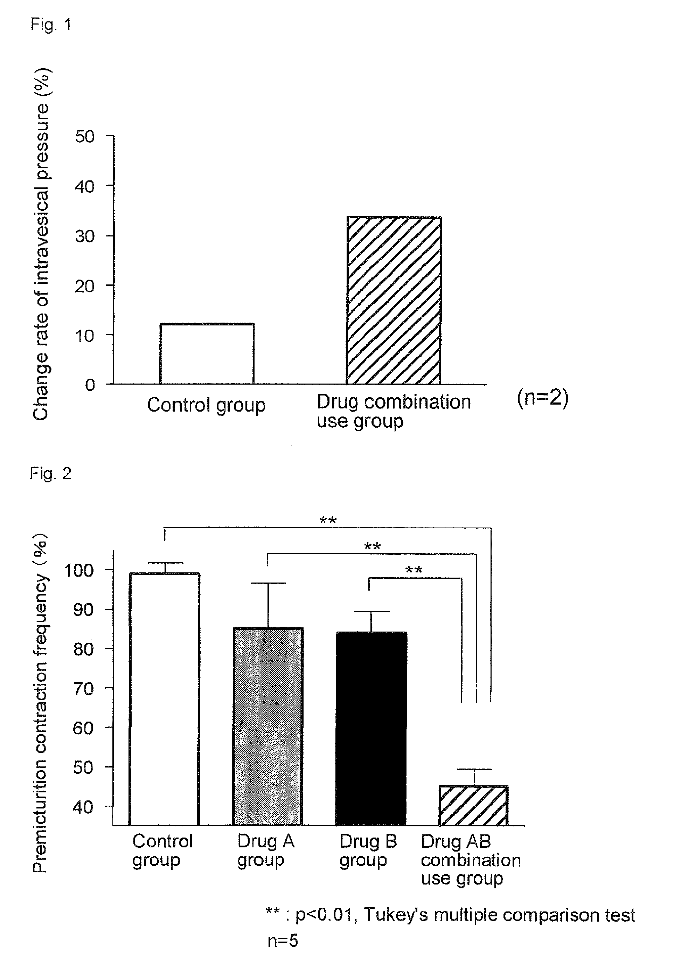

[Fig. 1] Fig. 1 shows the decreasing effects on intravesical pressure in each of the control group and the drug combination use group in Example 1.
[Fig. 2] Fig. 2 shows a frequency of premicturition contraction in each of the control group, the drug A group, the drug B group, and the drug AB combination use group in Example 2.

### Best Mode for Carrying Out the Invention

Hereinafter, the present invention is described in detail.
In the present specification, the term "improving lower urinary tract symptoms" refers to the improvement of lower urinary tract symptoms such as (1) voiding symptoms such as weak stream, fractionated urination, hesitancy, straining, and the like, (2) storage symptoms such as urinary frequency, nocturia, urinary urgency, and the like, and (3) postmicturition symptoms such as feeling of residual urine, postmicturition dribble, and the like. Although causes thereof are not particularly limited, for example, lower urinary tract symptoms associated with urethral stricture, neurogenic bladder, cystitis, bladder cancer, interstitial cystitis, prostate cancer, chronic prostatitis or the like, and idiopathic lower urinary tract symptoms whose pathogenic cause has not yet been specified, in an embodiment, lower urinary tract symptoms associated with benign prostatic hyperplasia, are mentioned.
The term "improving lower urinary tract symptoms associated with benign prostatic hyperplasia" refers to the improvement of lower urinary tract symptoms described in (1) to (3) above, in patients with benign prostatic hyperplasia.

(R)-2-(2-aminothiazol-4-yl)-4'-{2-[(2-hydroxy-2-phenylethyl)amino]ethyl}acetanilide or a pharmaceutically acceptable salt thereof, which is an active ingredient of the pharmaceutical composition of the present invention, can be easily obtained, for example, by the methods described in Pamphlet of International Publication No. WO 99/20607, methods apparent to those skilled in the art, or modified methods thereof.
Further, (R)-5-(2-{[2-(2-ethoxyphenoxy)ethyl]amino}propyl)-2-methoxybenzene-1-sulfonamide or a pharmaceutically acceptable salt thereof, which is an active ingredient of the pharmaceutical composition of the present invention, can be easily obtained, for example, by the methods described in US Patent No. 4,703,063, methods apparent to those skilled in the art, or modified methods thereof.

The term "a pharmaceutically acceptable salt thereof' in "(R)-2-(2-aminothiazol-4-yl)-4'-{2-[(2-hydroxy-2-phenylethyl)amino]ethyl}acetanilide or a pharmaceutically acceptable salt thereof" and in "(R)-5-(2-{[2-(2-ethoxyphenoxy)ethyl]amino}propyl)-2-methoxybenzene-1-sulfonamide or a pharmaceutically acceptable salt thereof' refers to the salts with the acids described in Pamphlet of International Publication No. WO 99/20607 or US Patent No. 4,703,063, respectively. Specifically, examples thereof include acid addition salts with inorganic acids such as hydrochloric acid, hydrobromic acid, hydroiodic acid, sulfuric acid, nitric acid, and phosphoric acid, or with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, and glutamic acid, salts with inorganic bases such as sodium, potassium, magnesium, calcium, and aluminum, or with organic bases such as methylamine, ethylamine, ethanolamine, lysine, and ornithine, salts with various amino acids such as acetylleucine and amino acid derivatives, ammonium salts, and the like.

Further, "a pharmaceutically acceptable salt" in "(R)-2-(2-aminothiazol-4-yl)-4'-{2-[(2-hydroxy-2-phenylethyl)amino]ethyl}acetanilide or a pharmaceutically acceptable salt thereof" and in "(R)-5-(2-{[2-(2-ethoxyphenoxy)ethyl]amino}propyl)-2-methoxybenzene-1-sulfonamide or a pharmaceutically acceptable salt thereof' may be various hydrates or solvates, and polymorphism, which are encompassed in the active ingredients of the pharmaceutical composition of the present invention. Further, a pharmaceutical composition containing a compound labeled with various radioactive or non-radioactive isotopes is also included in the present invention.

Embodiments of the present invention are shown below.

(1) Among the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof, of the present invention; the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof, wherein (R)-2-(2-aminothiazol-4-yl)-4'-{2-[(2-hydroxy-2-phenylethyl)amino]ethyl}acetanilide or a pharmaceutically acceptable salt thereof is (R)-2-(2-aminothiazol-4-yl)-4'-{2-[(2-hydroxy-2-phenylethyl)amino]ethyl}acetanilide.

(2) Among the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, of the present invention; the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, wherein (R)-5-(2-{[2-(2-ethoxyphenoxy)ethyl]amino}propyl)-2-methoxybenzene-1-sulfonamide or a pharmaceutically acceptable salt thereof is (R)-5-(2-{[2-(2-ethoxyphenoxy)ethyl]amino}propyl)-2-methoxybenzene-1-sulfonamide hydrochloride.

(3) Among the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof, of the present invention; the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof, wherein the amount of (R)-2-(2-aminothiazol-4-yl)-4'-{2-[(2-hydroxy-2-phenylethyl)amino]ethyl}acetanilide or a pharmaceutically acceptable salt thereof to be contained is from of 10 mg to 100 mg in terms of (R)-2-(2-aminothiazol-4-yl)-4'-{2-[(2-hydroxy-2-phenylethyl)ammo]ethyl}acetanilide. In another embodiment, the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof, wherein the amount of (R)-2-(2-aminothiazol-4-yl)-4'-{2-[(2-hydroxy-2-phenylethyl)amino]ethyl}acetanilide or a pharmaceutically acceptable salt thereof to be contained is 10 mg, 25 mg, 50 mg, or 100 mg in terms of (R)-2-(2-aminothiazol-4-yl)-4'-{2-[(2-hydroxy-2-phenylethyl)amino]ethyl}acetanilide.

(4) Among the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, of the present invention; the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, wherein the amount of (R)-5-(2-{[2-(2-ethoxyphenoxy)ethyl]amino}propyl)-2-methoxybenzene-1-sulfonamide or a pharmaceutically acceptable salt thereof to be contained is from 0.1 mg to 0.8 mg in terms of (R)-5-(2-{[2-(2-ethoxyphenoxy)ethyl]amino}propyl)-2-methoxybenzene-1-sulfonamide hydrochloride. In another embodiment, the pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof; wherein the amount of (R)-5-(2-{[2-(2-ethoxyphenoxy)ethyl]amino}propyl)-2-methoxybenzene-1-sulfonamide or a pharmaceutically acceptable salt thereof to be contained is 0.1 mg, 0.2 mg, 0.4 mg or 0.8 mg in terms of (R)-5-(2-{[2-(2-ethoxyphenoxy)ethyl]amino}propyl)-2-methoxybenzene-1-sulfonamide hydrochloride.

(5) The pharmaceutical composition or the pharmaceutical composition for improving lower urinary tract symptoms, for oral administration, the method for improving anterior lower urinary tract symptoms by oral administration, or the use for the manufacture of a pharmaceutical composition for oral administration for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof or the use for the manufacture of a pharmaceutical composition for oral administration for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof.

(6) Among the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, of the present invention; the pharmaceutical composition for improving lower urinary tract symptoms, the method for improving lower urinary tract symptoms, or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof, wherein the lower urinary tract symptoms are lower urinary tract symptoms associated with benign prostatic hyperplasia.

(7) The pharmaceutical composition or the pharmaceutical composition for improving lower urinary tract symptoms, the method for improving lower urinary tract symptoms, or the use for the manufacture of a pharmaceutical composition for improving lower urinary tract symptoms which is used in combination with (R)-2-(2-aminothiazol-4-yl)-4'-{2-[(2-hydroxy-2-phenylethyl)amino]ethyl}acetanilide or a pharmaceutically acceptable salt thereof or the use for the manufacture of a pharmaceutical composition for improving lower urinary tract symptoms which is used in combination with (R)-5-(2-{[2-(2-ethoxyphenoxy)ethyl]amino}propyl)-2-methoxybenzene-1-sulfonamide or a pharmaceutically acceptable salt thereof; which is a combination of two or more of (1) to (6) above.

The pharmaceutical composition of the present invention can be prepared by a generally used method, using (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, pharmaceutical carriers, excipients, and other additives, which are usually used for formulation. The administration can be any form of oral administration via tablets, pills, capsules, granules, powders, liquid preparations, or the like, or parenteral administration via injections such as intraarticular, intravenous, intramuscular, or the like, suppositories, eye drops, eye ointments, percutaneous liquid preparations, ointments, percutaneous patches, transmucosal liquid preparations, transmucosal patches, inhalations, or the like, and in a certain embodiment, oral administration can be mentioned.

As the solid composition for oral administration, tablets, powders, granules, or the like are used. In such a solid composition, the active ingredients are mixed with at least one inert excipient or the like. According to a conventional method, the composition may contain inert additives, for example, a lubricant, a disintegrator, a stabilizing agent, and a solubilizing aid. As occasion demands, the tablets or the pills may be coated with a sugar coating, or a film of gastric or enteric material.
The liquid composition for oral administration includes pharmaceutically acceptable emulsions, solutions, suspensions, syrups, elixirs, or the like, and contains a generally used inert diluent such as purified water or ethanol. In addition to the inert diluent, this liquid composition may contain an auxiliary agent such as a solubilizing agent, a moistening agent, and a suspending agent, a sweetener, a flavor, an aroma, and an antiseptic.

The injections for parenteral administration include sterile aqueous or non-aqueous liquid preparations, suspensions or emulsions. The aqueous solvent includes, for example, distilled water for injection or physiological saline. Examples of the non-aqueous solvent include propylene glycol, polyethylene glycol, plant oils such as olive oil, alcohols such as ethanol, Polysorbate 80 (Japanese Pharmacopeia), or the like. Such a composition may further contain a tonicity agent, an antiseptic, a moistening agent, an emulsifying agent, a dispersing agent, a stabilizing agent, or a solubilizing agent. These are sterilized, for example, by filtration through a bacteria retaining filter, blending of a bactericide, or irradiation. Additionally, these can also be used by preparing a sterile solid composition, and dissolving or suspending it in a sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like. Examples of the ointment bases or the lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, sorbitan sesquioleate, and the like.

As the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, hydrofluoroalkane, carbon dioxide, or the like, or other forms.

The combination use in the present invention may be administered simultaneously, or separately and successively or at a desired time interval. In the case of simultaneous administration, the preparation may be a blend, that is, a preparation containing both active ingredients in a single preparation, or alternatively, the separate preparations for each of the active ingredients may be prepared individually and then administered simultaneously.

### Examples

The pharmacological activity for the combination use of the active ingredients of the pharmaceutical composition according to the present invention was confirmed by the following experiments.

### Example 1: Effect on intravesical pressure in anesthetized rats at rest

### <Method>

Rats which were deprived of food and water overnight were used for the experiments. The rats were fixed to a dorsal position under pentobarbital anesthesia (50 mg/kg ip), and a catheter (PE-50) for administration of isoproterenol hydrochloride was inserted into the femoral vein. In addition, a midline incision was made in the upper abdominal wall, and a catheter (PE-100) for drug administration was inserted into the duodenum. Further, a catheter (PE-50) was inserted into the bladder from the external urethral orifice, and then ligated with the distal urethra. The intravesical pressure was measured by a pressure amplifier (AP-601G) through a pressure transducer (TP-400T) from the catheter inserted into the bladder. After the surgery, physiological saline was infused into the bladder through the catheter for measurement of the intravesical pressure until intravesical pressure became about 6 cmH₂O. After 5 minutes at which intravesical pressure was stabilized, vehicle or drug was administered into the duodenum.

Drugs for the two groups examined are as follows.
(1) "Control group":
   Physiological saline containing 5% dimethylacetamide and 5% cremophor 5 mL/kg id
(2) "Drug combination use group":
   Combination use of (R)-2-(2-aminothiazol-4-yl)-4'-{2-[(2-hydroxy-2-phenylethyl)amino]ethyl}acetanilide 2 mg/5 mL/kg id and (R)-5-(2-{[2-(2-ethoxyphenoxy)ethyl]amino}propyl)-2-methoxybenzene-1-sulfonamide hydrochloride 0.008 mg/5 mL/kg id

30 minutes after the drug administration, isoproterenol hydrochloride (0.01 mg/kg iv) as a positive control was administered. The minimum value of intravesical pressure during 5 minutes prior to the drug administration was taken as a pre-administration value, and the minimum value of intravesical pressure during 30 minutes after drug administration was taken as a post-administration value. Effects of the drug were evaluated with change rates (%) of the post-administration value when the amount of the change between the pre-administration value and the minimum value of intravesical pressure decreased by the administration of isoproterenol hydrochloride was taken as 100.

In addition, the dose of each drug was set based on the following grounds.
(a) (R)-2-(2-aminothiazol-4-yl)-4'-{2-[(2-hydroxy-2-phenylethyl)amino]ethyl}acetanilide:
   A value obtained by dividing a dose of 100 mg, which is expected as the highest dose among the doses currently used in clinical trials, by 50. Accordingly, the dose of 2 mg(/5 mL)/kg used in the above experiment corresponds to a dose per unit weight when an adult weight is assumed to be 50 kg.
(b) (R)-5-(2-{[2-(2-ethoxyphenoxy)ethyl]amino}propyl)-2-methoxybenzene-1-sulfonamide hydrochloride:
   A value obtained by dividing an ordinary dose of 0.4 mg currently used in clinical trials by 50. Accordingly, the dose of 0.008 mg(/5 mL)/kg used in the above experiment corresponds to a dose per unit weight when an adult weight is assumed to be 50 kg.

### <Results>

In rats, the decrease (change rate) in intravesical pressure in the control group was 12.1 %. On the other hand, the decrease (change rate) in intravesical pressure in the drug combination use group was 33.7%, which is higher than that of the control group.

### Example 2: Effect on premicturition contraction in rats with bladder outlet obstruction

### <Method>

The rats were fixed to a dorsal position under pentobarbital anesthesia (50 mg/kg ip), and a catheter (PE-20) was inserted into the bladder from the external urethral orifice. A midline incision was made in the lower abdominal wall, and the proximal urethra, together with the catheter, was tied at two sites with a silk suture (5-0), then, the catheter was removed and the surgical wound was sutured, and then ampicillin (150 mg/kg sc) as an antibiotic was administered to the animals which were then housed in a breeding cage.

On the 11th to 12th day after the surgery, a midline incision was made in the lower abdominal wall under pentobarbital anesthesia (50 mg/kg ip), and the silk sutures tying the urethra were removed. The bladder was exposed, followed by drainage of urine, and a catheter (PE-50) was inserted into the bladder from the apical part the bladder and fixed. The other end of this catheter was lead to the outside from the posterior cervical region and then the surgical wound was sutured. In addition, a catheter (PE-50) for the drug administration was inserted into the jugular vein, and then the other end thereof was lead to the outside from the posterior cervical region. Ampicillin (150 mg/kg sc) was administered to the animals which were then returned to a breeding cage.

Two to three days later, the rats were retained in a Bollman cage. The catheter inserted into the bladder was connected to a three-way stopcock valve, and one end was connected to a syringe pump (STC-525) and micturition reflex was induced by continuous infusion of physiological saline [3.6 to 12 mL/h (adjusted to achieve a voiding interval of 20 to 40 minutes)]. In addition, the other end was connected to a pressure transducer (TP-400T), and intravesical pressure was measured with a pressure amplifier (AP-601G). The premicturition contraction frequency [evaluated for 10 minutes prior to micturition reflex (maximum intravesical pressure); a contraction showing a drop of 1 cmH₂O or more after a rise of 2 cmH₂O or more was evaluated as one time] after the stabilization of micturition reflex by continuous infusion of physiological saline for 2 hours or more was taken as a pre-administration value. Vehicle or each drug was intravenously administered after intravesical pressure was lowered to a baseline, and the premicturition contraction frequency in the first micturition reflex was taken as a post-administration value, and evaluated by a ratio (%) relative to the pre-administration value.

Drugs for the four groups examined are as follows. Evaluation was carried out using 5 animals for each group.
(1) "control group":
   Physiological saline containing 5% dimethylacetamide and 5% cremophor 1 mL/kg iv
(2) "Drug A group":
   (R)-2-(2-aminothiazol-4-yl)-4'-{2-[(2-hydroxy-2-phenylethyl)amino]ethyl}acetanilide 0.3 mg/mL/kg iv
(3) "Drug B group":
   (R)-5-(2-{[2-(2-ethoxyphenoxy)ethyl]amino}propyl)-2-methoxybenzene-1-sulfonamide hydrochloride 0.001 mg/mL/kg iv
(4) "Drug AB combination use group":
   Combination use of (R)-2-(2-aminothiazol-4-yl)-4'-{2-[(2-hydroxy-2-phenylethyl)amino]ethyl}acetanilide 0.3 mg/mL/kg iv and (R)-5-(2-{[2-(2-ethoxyphenoxy)ethyl]amino}propyl)-2-methoxybenzene-1-sulfonamide hydrochloride 0.001 mg/mL/kg iv

In addition, the dose of each drug was set based on the following grounds.
(a) (R)-2-(2-aminothiazol-4-yl)-4'-{2-[(2-hydroxy-2-phenylethyl)amino]ethyl}acetanilide:
   The dose exhibiting about 15% inhibition of the premicturition contraction frequency, at which a significant inhibition of the premicturition contraction frequency is not recognized as a single use (0.3 mg(/mL)/kg iv).
(b) (R)-5-(2-{[2-(2-ethoxyphenoxy)ethyl]amino}propyl)-2-methoxybenzene-1-sulfonamide hydrochloride:
   The dose exhibiting about 15% inhibition of the premicturition contraction frequency, at which a significant inhibition of the premicturition contraction frequency is not recognized as a single use (0.001 mg(/mL)/kg iv).

### <Results>

In rats, the ratio of the premicturition contraction frequency relative to the pre-administration value in the control group was 98.9%. On the other hand, the ratios for the drug A group, the drug B group, and the drug AB combination use group was 85.4%, 84.8%, and 45.3%, respectively, and the drug AB combination use group significantly inhibited the premicturition contraction compared to any other groups.

From the above results, it was demonstrated that, by a combination use of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof as the active ingredients of the pharmaceutical composition of the present invention, or a combined agent supposed to be used simultaneously in combination, excellent improving effects on storage symptoms, for which an α₁-adrenoceptor antagonist alone was considered hitherto to be insufficient, were exhibited. Accordingly, it was demonstrated that among lower urinary tract symptoms, storage symptoms as well as voiding symptoms are sufficiently improved by a combination use of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, which are α₁-adrenoceptor antagonists, or a combined agent supposed to be used simultaneously in combination.

The pharmaceutical composition according to the present invention can be prepared, for example, in accordance with the following formula, and the effects thereof can be evaluated in accordance with the methods described in the above Examples, or improved methods or modified methods thereof.

### Example 3: Preparation of the film-coated tablet according to the present invention

Hydroxypropyl methylcellulose 2910 (200 parts) is dissolved under stirring in water (1800 parts) with an air motor agitator (AM-GC-1, manufactured by Chuo-Rika Machine) to prepare a binder solution. (R)-5-(2-{[2-(2-ethoxyphenoxy)ethyl]amino}propyl)-2-methoxybenzene-1-sulfonamide hydrochloride (10 parts), (R)-2-(2-aminothiazol-4-yl)-4'-(2-[(2-hydroxy-2-phenylethyl)amino]ethyl}acctanilide (1250 parts), lactose (2490 parts), corn starch (750 parts), and Carmellose calcium (250 parts) are mixed with a mixer (type DC, manufactured by Astellas Pharma Inc.). The mixture is introduced into a fluidized bed granulator (WSG-5, manufactured by Powrex Co., Ltd.), and the above binder solution is sprayed for granulation and then dried to obtain a granulated product. Magnesium stearate (12 parts) is added to the dried granulated product (1188 parts), and mixed with a mixer. The mixture is compressed with a pestle and mortar having a diameter of 8 mm, using a rotary tableting machine (HTP-22, manufactured by Hata Iron Works, Co., Ltd.) to obtain a tablet weighing 200 mg. Using an aerated coating machine (Hi-Coater HCT-48, manufactured by Freund Industry, Co., Ltd.), this tablet (3000 parts) is sprayed and coated with a solution prepared by dissolving/dispersing hydroxypropyl methylcellulose 2910 (100.5 parts), Macrogol 6000 (18.8 parts), talc (30.2 parts), titanium oxide (12.5 parts) and red iron sesquioxide (0.04 parts) in water (1457.6 parts), until the coating component becomes 2.7% of the tablet weight, to obtain a film-coated tablet.

### Example 4: Preparation of the capsule agent according to the present invention (1)

(R)-5-(2-{[2-(2-ethoxyphenoxy)ethyl]amino}propyl)-2-methoxybenzene-1-sulfonamide hydrochloride (2.5 parts), (R)-2-(2-aminothiazol-4-yl)-4'-{2-[(2-hydroxy-2-phenylethyl)amino]ethyl}acetanilide (250 parts), and lactose (4747.5 parts) are mixed with a mixer (type DC, manufactured by Astellas Pharma Inc.). 200 mg of this mixture is filled into a capsule (gelatin hard capsule No. 4, manufactured by Capsugel Inc.) with a small-scale capsule filling machine (Profill, manufactured by Capsugel Inc.),to obtain a capsule agent.

### Example 5: Preparation of the capsule agent according to the present invention (2)

(R)-5-(2-{[2-(2-ethoxyphenoxy)ethyl]amino}propyl)-2-methoxybenzene-1-sulfonamide hydrochloride (5 parts), (R)-2-(2-aminothiazol-4-yl)-4'-{2-[(2-hydroxy-2-phenylethyl)amino]ethyl}acetanilide (625 parts), and lactose (4370 parts) are mixed with a mixer (type DC, manufactured by Astellas Pharma Inc.). 200 mg of this mixture is filled into a capsule (gelatin hard capsule No. 4, manufactured by Capsugel Inc.) with a small-scale capsule filling machine (Profill, manufactured by Capsugel Inc.), to obtain a capsule agent.

### Example 6: Preparation of the capsule agent according to the present invention (3)

(R)-5-(2-({[2-(2-ethoxyphenoxy)ethyl]amino}propyl)-2-methoxybenzene-1-sulfonamide hydrochloride (14 parts), (R)-2-(2-aminothiazol-4-yl)-4'-{2-[(2-hydroxy-2-phenylethyl)amino]ethyl}acetanilide (1750 parts), and lactose (2961 parts) are mixed with a mixer (type DC, manufactured by Astellas Pharma Inc.). 270 mg of this mixture is filled into a capsule (gelatin hard capsule No. 3, manufactured by Capsugel Inc.) with a small-scale capsule filling machine (Profill, manufactured by Capsugel Inc.), to obtain a capsule agent.

### Industrial Applicability

A pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof as active ingredients can be used as a pharmaceutical composition for improving lower urinary tract symptoms.

## Claims

1. A pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, as active ingredients.

2. The pharmaceutical composition of claim 1, wherein the (R)-2-(2-aminothiazol-4-yl)-4'-{2-[(2-hydroxy-2-phenylethyl)amino]ethyl}acetanilide or a pharmaceutically acceptable salt thereof is (R)-2-(2-aminothiazol-4-yl)-4'-{2-[(2-hydroxy-2-phenylethyl)amino]ethyl}acetanilide.

3. The pharmaceutical composition of claim 1 or 2, wherein the (R)-5-(2-{[2-(2-ethoxyphenoxy)ethyl]amino}propyl)-2-methoxybenzene-1-sulfonamide or a pharmaceutically acceptable salt thereof is (R)-5-(2-{[2-(2-ethoxyphenoxy)ethyl]amino}propyl)-2-methoxybenzene-1-sulfonamide hydrochloride.

4. The pharmaceutical composition of any one of claims 1 to 3, further comprising a pharmaceutically acceptable excipient.

5. A pharmaceutical composition comprising (R)-2-(2-aminothiazol-4-yl)-4'-{2-[(2-hydroxy-2-phenylethyl)amino]ethyl]acetanilide or a pharmaceutically acceptable salt thereof at an amount of 10 mg to 100 mg in terms of (R)-2-(2-aminothiazol-4-yl)-4'-{2-[(2-hydroxy-2-phenylethyl)amino]ethyl}acetanilide and (R)-5-(2-{[2-(2-ethoxyphenoxy)ethyl]amino}propyl)-2-methoxybenzene-1-sulfonamide or a pharmaceutically acceptable salt thereof at an amount of 0.1 mg to 0.8 mg in terms of (R)-5-(2-{[2-(2-ethoxyphenoxy)ethyl]amino}propyl)-2-methoxybenzene-1-sulfonamide hydrochloride, as active ingredients.

6. The pharmaceutical composition of claim 5, comprising 10 mg to 100 mg of (R)-2-(2-aminothiazol-4-yl)-4'-{2-[(2-hydroxy-2-phenylethyl)amino]ethyl}acetanilide and 0.1 mg to 0.8 mg of (R)-5-(2-{[2-(2-ethoxyphenoxy)ethyl]amino}propyl)-2-methoxybenzene-1-sulfonamide hydrochloride.

7. The pharmaceutical composition of any one of claims 1 to 6, which is a pharmaceutical composition for improving lower urinary tract symptoms.

8. The pharmaceutical composition of any one of claims 1 to 7, which is a preparation for oral administration.

9. A method for improving lower urinary tract symptoms, comprising administering effective amounts of each of (R)-2-(2-aminothiazol-4-yl)-4'-(2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof.

10. The method for improving of claim 9, comprising administering effective amounts of each of (R)-2-(2-aminothiazol-4-yl)-4'-{2-[(2-hydroxy-2-phenylethyl)amino]ethyl}acetanilide or a pharmaceutically acceptable salt thereof and (R)-5-(2-{[2-(2-ethoxyphenoxy)ethyl]amino}propyl)-2-methoxybenzene-1-sulfonamide or a pharmaceutically acceptable salt thereof, simultaneously or at a time interval.

11. Use of (R)-5-(2-{[2-(2-ethoxyphenoxy)ethyl]amino}propyl)-2-methoxybenzene-1-sulfonamide or a pharmaceutically acceptable salt thereof, for the manufacture of a pharmaceutical composition for improving lower urinary tract symptoms, which is used in combination with (R)-2-(2-aminothiazol-4-yl)-4'-{2-[(2-hydroxy-2-phenylethyl)amino]ethyl}acetanilide or a pharmaceutically acceptable salt thereof.

12. Use of (R)-2-(2-aminothiazol-4-yl)-4'-{2-[(2-hydroxy-2-phenylethyl)amino]ethyl}acetanilide or a pharmaceutically acceptable salt thereof, for the manufacture of a pharmaceutical composition for improving lower urinary tract symptoms, which is used in combination with (R)-5-(2-{[2-(2-ethoxyphenoxy)ethyl]amino}propyl)-2-methoxybenzene-1-sulfonamide or a pharmaceutically acceptable salt thereof

13. A pharmaceutical composition comprising (R)-2-(2-aminothiazol-4-yl)-4'-{2-[(2-hydroxy-2-phenylethyl)amino]ethyl}acetanilide or a pharmaceutically acceptable salt thereof as an active ingredient, which is used in combination with (R)-5-(2-{[2-(2-ethoxyphenoxy)ethyl]amino}propyl)-2-methoxybenzene-1-sulfonamide or a pharmaceutically acceptable salt thereof.

14. A pharmaceutical composition comprising (R)-5-(2-{[2-(2-ethoxyphenoxy)ethyl]amino}propyl)-2-methoxybenzene-1-sulfonamide or a pharmaceutically acceptable salt thereof as an active ingredient, which is used in combination with (R)-2-(2-aminothiazol-4-yl)-4'-{2-[(2-hydroxy-2-phenylethyl)amino]ethyl}acetanilide or a pharmaceutically acceptable salt thereof.
